# EUROPEAN PATENT APPLICATION

(11) **EP 2 875 783 A1**
(43) Date of publication of application: **27.05.2015**
(21) Application number: 14193632.8
(22) Date of filing: 18.11.2014
(51) Int. Cl.: A61B 8/00, B06B 1/06, G10K 11/02, G10K 11/30

(54) **Ultrasonic device and manufacturing method thereof, electronic equipment, and ultrasonic image device**

(30) Priority: 20.11.2013 JP 2013240277
(71) Applicant: Seiko Epson Corporation, Shinjuku-ku, Tokyo 163-0811 (JP)
(72) Inventor: Kiyose, Kanechika, Nagano, 392-8502 (JP)
(74) Representative: Hoffmann Eitle

(57) **Abstract**

An ultrasonic device (17) includes a substrate (44), an acoustic adjustment layer (51), an acoustic lens (52) and a structural member (53). The substrate has an element array (22) including a plurality of thin film ultrasonic transducer elements (28) arranged in an array form. The acoustic adjustment layer covers the element array. The acoustic lens is arranged above the acoustic adjustment layer. The structural member is in contact with the acoustic lens and fixed to the substrate. The structural member has a modulus of rigidity greater than a modulus of rigidity of the acoustic lens.

## Description

### BACKGROUND

### Technical Field

The present invention relates to an ultrasonic device and the manufacturing method thereof, as well as to a probe, electronic equipment, an ultrasonic image device or the like that uses those.

### Related Art

As disclosed in Japanese Unexamined Patent Publication No. 2008-193357, an ultrasonic image device such as an ultrasonic diagnostic device is generally known. The ultrasonic image device is equipped with a plurality of ultrasonic transducer elements arranged in array form. The ultrasonic transducer element is formed with a so-called cMUT (electrostatic capacity type) oscillator. An acoustic lens is covered on the array of ultrasonic transducer elements. The acoustic lens is adhered to the array of ultrasonic transducer elements using an adhesive agent. The acoustic lens is formed form silicone rubber.

### SUMMARY

When matching with a living body, an acoustic lens has acoustic impedance close to that of the living body. Therefore, the acoustic lens has the same level of softness as the living body. When an acoustic lens pressed against a living body is moved along the surface of the living body, the acoustic lens is exposed to shear force, and there is a risk of the acoustic lens undergoing shear deformation. For example, as disclosed in Japanese Unexamined Patent Publication No. 2008-193357, when the acoustic lens is fit into an ultrasonic probe case and aligned, shear force acts on the acoustic lens from the case according to the fitting tolerance, and it is easy for shear deformation of the acoustic lens to be caused.

In light of that, there was a desire for an ultrasonic device that could sufficiently suppress shear deformation of the acoustic lens in relation to shear force.
(1) An ultrasonic device according to one aspect includes a substrate, an acoustic adjustment layer, an acoustic lens and a structural member. The substrate has an element array including a plurality of thin film ultrasonic transducer elements arranged in an array form. The acoustic adjustment layer covers the element array. The acoustic lens is arranged above the acoustic adjustment layer. The structural member is in contact with the acoustic lens and fixed to the substrate. The structural member has a modulus of rigidity greater than a modulus of rigidity of the acoustic lens.

When the acoustic lens is pressed against a living body and moved along the surface of the living body, the acoustic lens is exposed to shear force from a specific direction. At this time, the acoustic lens is received by the structural member in the shearing direction. Even when shear force is imparted to the acoustic lens, it is possible to prevent shear deformation of the acoustic lens. In this way, with the ultrasonic device, it is possible to sufficiently suppress shear deformation of the acoustic lens in relation to the shear force.
(2) With the ultrasonic device, the acoustic adjustment layer is preferably in contact with the structural member, and is fixed to the substrate. The acoustic adjustment layer is received by the structural member in the shearing direction. Therefore, even when shear force is imparted to the acoustic adjustment layer, it is possible to prevent shear deformation of the acoustic adjustment layer. Prevention of shear deformation of the acoustic adjustment layer can greatly contribute to the suppression of shear deformation of the acoustic lens.
(3) With the ultrasonic device, the structural member preferably includes a pair of side surfaces that extend in parallel with a generatrix of the acoustic lens, the side surfaces of the structural member being respectively in contact with side surfaces of the acoustic lens as the acoustic lens is sandwiched by the structural member. A partial cylindrical surface formed by a generatrix parallel to one center line is defined on the acoustic lens. The partial cylindrical surface is used to converge ultrasonic waves. The focus position of the ultrasonic waves is determined by the partial cylindrical surface. The side surfaces of the structural member sandwich the acoustic lens in the direction intersecting with the generatrix, so shear deformation of the acoustic lens is prevented in the direction intersecting with the generatrix. In this way, it is possible to maintain the focus position of the acoustic lens. When the acoustic lens has shear deformation in the direction intersecting the generatrix of the acoustic lens, there is a risk of displacement of the focus position of the acoustic lens occurring. When the focus position is displaced, it is not possible to draw an accurate ultrasonic image.
(4) With the ultrasonic device, the acoustic adjustment layer preferably has a pair of side surfaces that extend flush with the side surfaces of the acoustic lens, respectively. During production of the ultrasonic device, a solid acoustic lens is adhered to the element array using a fluid resin material. The resin material is hardened to form the acoustic adjustment layer. At this time, on the substrate, at the same time that the acoustic lens edge is being aligned, a physical surface is formed that dams the flow of the resin material. The acoustic lens is accurately aligned to the element array. When the resin material hardens, the side surface of the acoustic lens and the side surface of the acoustic adjustment layer are flush with each other.
(5) With the ultrasonic device, the structural member preferably includes a pair additional side surfaces that extend in a direction intersecting with the generatrix of the acoustic lens, the additional side surfaces of the structural member being respectively in contact with additional side surfaces of the acoustic lens as the acoustic lens is sandwiched by the structural member. Here, the side surface of the structural member sandwiches the acoustic lens in the direction along the generatrix of the acoustic lens, so shear deformation of the acoustic lens is prevented in the direction along the generatrix. This kind of shear deformation prevention greatly contributes to the prevention of displacement of the focus position.
(6) With the ultrasonic device, the acoustic adjustment layer preferably has a pair of additional side surfaces that extend flush with the additional side surfaces of the acoustic lens, respectively. During production of the ultrasonic device, a solid acoustic lens is adhered to the element array using a fluid resin material. The resin material is hardened to form the acoustic adjustment layer. At this time, on the substrate, at the same time that the acoustic lens edge is being aligned, a physical surface is formed that dams the flow of the resin material. The acoustic lens is accurately aligned to the element array. When the resin material hardens, the side surface of the acoustic lens and the side surface of the acoustic adjustment layer are flush with each other.
(7) With the ultrasonic device, the acoustic lens preferably has a pair of additional side surfaces that extend in a direction intersecting the generatrix of the acoustic lens, the acoustic adjustment layer preferably includes a pair of additional side surfaces that extend in the direction intersecting the generatrix of the acoustic lens, and the additional side surfaces of the acoustic lens and the additional side surfaces of the acoustic adjustment layer are preferably flush with a pair of side surfaces of the substrate, respectively. For formation of the acoustic adjustment layer, the edge of the acoustic lens is aligned to the edge of the substrate, and at the same time, a physical surface is formed that dams the flow of the resin material. The physical surface regulates the movement of the acoustic lens in the direction parallel to the generatrix of the acoustic lens. The edge of the acoustic lens is matched to the edge of the substrate. When the resin material is hardened, the side surface of the acoustic lens and the side surface of the acoustic adjustment layer are flush with the side surface of the substrate.
(8) The ultrasonic device preferably further includes a flexible printed wiring board coupled to the substrate at an outside of an outline of the acoustic adjustment layer in a plan view along a thickness direction of the substrate, and a conductor member disposed on the substrate. At least a part of the structural member is preferably arranged on the conductor member between the acoustic adjustment layer and the flexible printed wiring board. The element array and the flexible printed wiring board are electrically connected to each other by a conductor member on the substrate. The conductor member is covered by the structural member between the acoustic adjustment layer and the flexible printed wiring board, so exposure of the conductor member is avoided. In this way, the conductor member on the substrate is protected.
(9) With the ultrasonic device, at least a part of the structural member is preferably arranged on the flexible printed wiring board. The structural member can reinforce the fixing strength of the flexible printed wiring board.
(10) The ultrasonic device can be used incorporated in a probe. At this time, the probe can be equipped with the ultrasonic device, and a case to support the ultrasonic device.
(11) With the probe, the structural member is preferably fixed to the case. During production of the probe, the ultrasonic device is housed inside the case. The structural member prevents movement of the ultrasonic device within the case. The acoustic lens is fixed reliably in the case.
(12) The ultrasonic device can be used incorporated in an electronic equipment. At this time, the electronic equipment can be equipped with the ultrasonic device, and a processing unit connected to the ultrasonic device, configured to process the output of the ultrasonic device.
(13) The ultrasonic device can also be used incorporated in an ultrasonic image device. At this time, the ultrasonic image device can be equipped with the ultrasonic device, a processing unit connected to the ultrasonic device for processing the output of the ultrasonic device and generating an image, and a display device for displaying the image.
(14) An ultrasonic device manufacturing method according to another aspect includes: arranging a masking material on a substrate having an element array including a plurality of thin film ultrasonic transducer elements arranged in an array form so that both sides of an area on which the element array is arranged in a plan view along a thickness direction of the substrate are covered by the masking material; arranging an acoustic adjustment layer and an acoustic lens on the element array between the masking material; removing the masking material; and forming a structural member in contact with the acoustic lens and fixed to the substrate, the structural member having a modulus of rigidity greater than a modulus of rigidity of the acoustic lens. In this way, the ultrasonic device described above can be produced.
(15) The ultrasonic device manufacturing method preferably further includes, after the removing of the masking material, coupling a flexible printed wiring board to the substrate between edges of the area and edges of the substrate in the both sides of the area. The forming of the structural member preferably includes flowing a resin material having fluidity in between the acoustic adjustment layer and the flexible printed wiring board, and hardening the resin material to form the structural member.

The element array and the flexible printed wiring board are electrically connected to each other by a conductor member on the substrate. The conductor member is covered by the structural member between the acoustic adjustment layer and the flexible printed wiring board, so exposure of the conductor member is avoided. When forming the structural member, resin material is flowed into the space between the acoustic adjustment layer and the flexible printed wiring board. The resin material can reliably cover the conductor member. On the other hand, for example when the dropped resin material droplets are pushed and spread by another member and the resin material fills in the space, it is not possible for the resin material to sufficiently reach every corner of the space.
(16) With the ultrasonic device manufacturing method, the arranging of the acoustic lens preferably includes using the masking material to position the acoustic lens from both sides. With the work of the masking material, it is possible to align the acoustic lens to the element array with high precision.
(17) With the ultrasonic device manufacturing method, the arranging of the acoustic lens preferably includes placing the acoustic lens within an opening formed in the masking material to position the acoustic lens from four directions on the acoustic lens. With the work of the masking material, it is possible to align the acoustic lens to the element array with high precision.
(18) With the ultrasonic device manufacturing method, the arranging of the acoustic adjustment layer preferably includes flowing a resin material having fluidity into the opening and controlling a thickness of the resin material by a thickness of the masking material. The opening demarcates a frame enclosing the element array on the substrate. It is possible to flow the resin material of the acoustic adjustment layer in the frame. The flow of the resin material is dammed. In this way, the shape of the acoustic adjustment layer is arranged. In this way, it is possible to determine the thickness of the acoustic adjustment layer.

### BRIEF DESCRIPTION OF THE DRAWINGS

Referring now to the attached drawings which form a part of this original disclosure:
FIG. 1 is an external view schematically showing one example of the electronic equipment of an embodiment, specifically, the ultrasonic diagnostic device.
FIG. 2 is an enlarged front view of the ultrasonic probe.
FIG. 3 is an enlarged plan view of the ultrasonic device of the first embodiment.
FIG. 4 is a cross section view along line A-A of FIG. 3.
FIG. 5 is a perspective view of the ultrasonic device.
FIG. 6 is a cross section view along line B-B of FIG. 5.
FIG. 7 is a drawing showing the manufacturing method of the ultrasonic device, and is a partial enlarged cross section view schematically showing the masking member formed on the substrate.
FIG. 8 is a drawing showing the manufacturing method of the ultrasonic device, and is a partial enlarged cross section view schematically showing the resin material flowed in on the element array.
FIG. 9 is a drawing showing the manufacturing method of the ultrasonic device, and is a partial enlarged cross section view schematically showing the acoustic lens arranged in the opening of the masking material.
FIG. 10 is a drawing showing the manufacturing method of the ultrasonic device, and is a partial enlarged cross section view schematically showing the first wiring board and the second wiring board coupled to the substrate after the masking material is removed.
FIG. 11 is a drawing showing the manufacturing method of the ultrasonic device, and is a partial enlarged cross section view schematically showing the protective film formed on the substrate.
FIG. 12 is a perspective view schematically showing the ultrasonic device of a first modification example corresponding to FIG. 5.
FIG. 13 is a vertical cross section view schematically showing the ultrasonic device of a second modification example.

### DETAILED DESCRIPTION OF EXEMPLARY EMBODIMENTS

Hereafter, we will describe an embodiment of the present invention while referring to the attached drawings. This embodiment described hereafter does not unduly limit the contents of the present invention noted in the scope of patent claims, and all of the structures described with this embodiment are not absolutely necessary as means for solving of the present invention.

### (1) Overall Configuration of the Ultrasonic Diagnostic Device

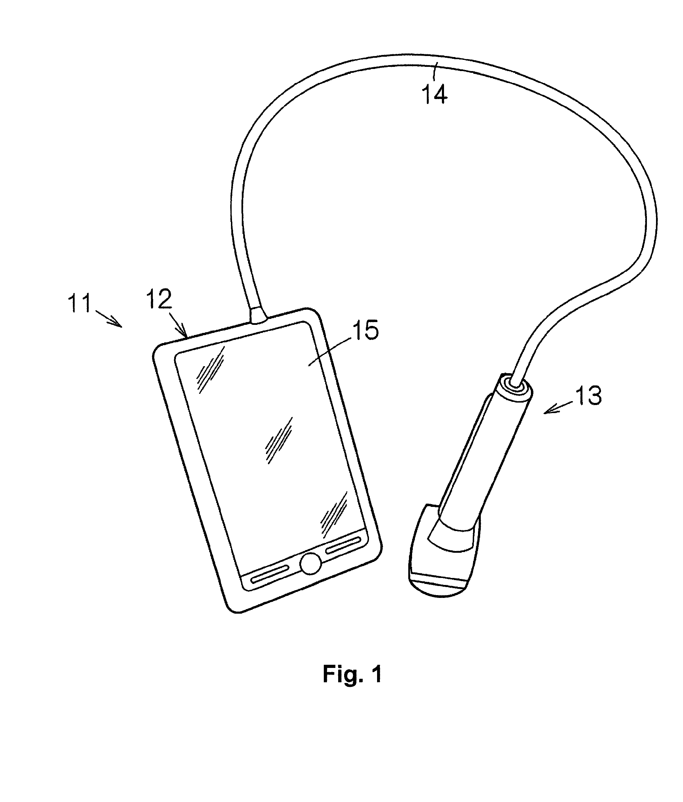

FIG. 1 schematically shows one example of electronic equipment of an embodiment of the present invention, specifically, the configuration of an ultrasonic diagnostic device (ultrasonic image device) 11. The ultrasonic diagnostic device 11 is equipped with a device terminal (processing unit) 12 and an ultrasonic probe (probe) 13. The device terminal 12 and the ultrasonic probe 13 are connected to each other by a cable 14. The device terminal 12 and the ultrasonic probe 13 exchange electronic signals through the cable 14. A display panel (display device) 15 is incorporated in the device terminal 12. The screen of the display panel 15 is exposed on the surface of the device terminal 12. With the device terminal 12, an image is generated based on the ultrasonic waves detected by the ultrasonic probe 13. The detected results put into image form are displayed on the screen of the display panel 15.

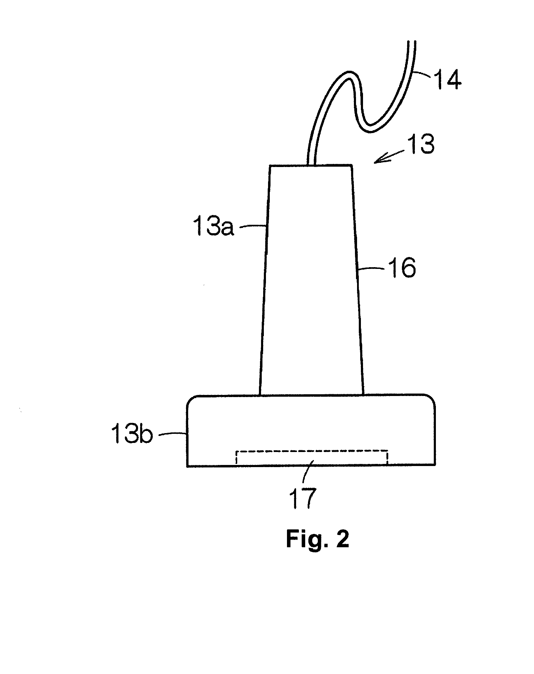

As shown in FIG. 2, the ultrasonic probe 13 has a case 16. Inside the case 16 is housed an ultrasonic device 17. The surface of the ultrasonic device 17 can be exposed on the surface of the case 16. The ultrasonic device 17 outputs ultrasonic waves from the surface and receives reflected waves of the ultrasonic waves. In addition, the ultrasonic probe 13 can be equipped with a probe head 13b linked so as to be freely detachable with a probe main unit 13a. At this time, the ultrasonic device 17 can be incorporated inside the case 16 of the probe head 13b.

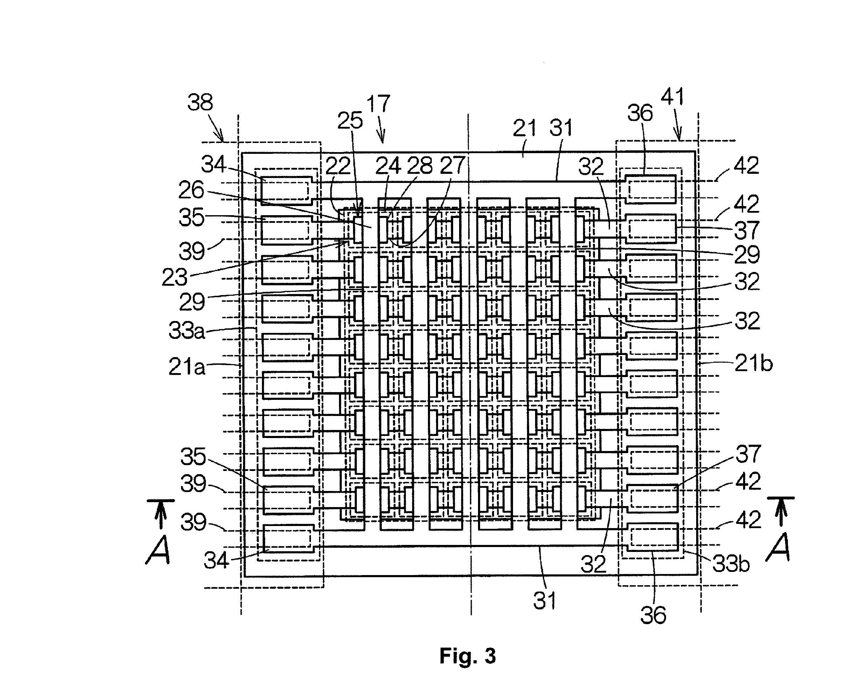

FIG. 3 schematically shows a plan view of the ultrasonic device 17. The ultrasonic device 17 is equipped with a base (base material) 21. An element array 22 is formed on the base 21. The element array 22 is constituted with an array of ultrasonic transducer elements (hereafter called "elements") 23 arranged in array form. The array is formed in a matrix of a plurality of rows and a plurality of columns. In addition, it is possible to establish a zigzag arrangement for the array. With a zigzag arrangement, the even numbered row element 23 group can be skewed by a column pitch of 1/2 in relation to the odd numbered row element 23 group. The number of elements of one of the odd numbered row and the even numbered row can be one less than the number of elements of the other.

Each individual element 23 is equipped with a vibration film 24. FIG. 3 depicts the outline of the vibration film 24 with a dotted line in a plan view in the direction orthogonal to the film surface of the vibration film 24 (plan view along the substrate thickness direction). A piezoelectric element 25 is formed on the top of the vibration film 24. The piezoelectric element 25 is constituted with an upper electrode 26, a lower electrode 27, and a piezoelectric film 28. The piezoelectric film 28 is sandwiched between the upper electrode 26 and the lower electrode 27 for each element 23. These are overlapped in the sequence of the lower electrode 27, the piezoelectric film 28, and the upper electrode 26. The ultrasonic device 17 is constituted as one ultrasonic transducer element chip (substrate).

A plurality of first conductors (signal lines) 29 (one example of a conductor member) are formed on the surface of the base 21. The first conductors 29 extend mutually in parallel to the column direction of the array. One first conductor 29 is allocated per column of elements 23. One first conductor 29 is connected in common to the piezoelectric film 28 of elements 23 aligned in the column direction of the array. The first conductor 29 has the upper electrode 26 formed for each individual element 23. Both ends of the first conductor 29 are respectively connected to a pair of lead-out wires 31. The lead-out wires 31 extend mutually in parallel in the row direction of the array. Therefore, all of the first conductors 29 have the same length. In this way, the upper electrodes 26 are connected in common to the elements 23 of the entire matrix. The first conductors 29 can be formed using iridium (Ir), for example. Other conductive materials can also be used for the first conductors 29.

A plurality of second conductors 32 (one example of a conductor member) are formed on the surface of the base 21. The second conductors 32 extend mutually in parallel to the row direction of the array. One second conductor 32 is allocated per row of elements 23. One second conductor 32 is arranged in common with the piezoelectric film 28 of the elements 23 aligned in the row direction of the array. The second conductor 32 has the lower electrode 27 formed for each individual element 23. For example, a laminated film of titanium (Ti), iridium

(Ir), platinum (Pt), and titanium (Ti) can be can be used for the second conductor 32. However, it is also possible to use other conductive materials for the second conductor 32.

The energization of the elements 23 can be switched for each row. Linear scanning or sector scanning is realized according to this energization switching. Since one row of elements 23 output ultrasonic waves simultaneously, it is possible to determine the number of rows, specifically, the number of columns of the array, according to the ultrasonic wave output level. The number of columns can be set to approximately 10 to 15 columns, for example. This is abbreviated in the drawing with five columns depicted. The number of rows of the array can be determined according to the expansion of the scan range. The number of rows can be set to 128 rows or 256 rows, for example. This is abbreviated in the drawing with eight rows depicted. The role of the upper electrodes 26 and the lower electrodes 27 can also be interchanged. Specifically, while the lower electrodes are connected in common to the elements 23 of the entire matrix, the upper electrodes can be connected to the elements 23 in common for each row of the array.

The outline of the base 21 has a first side 21 a and a second side 21b facing opposite, partitioned by a pair of straight lines that are mutually parallel. One line of first terminal arrays 33a is arranged between the first side 21a and the element array 22 outline. One line of second terminal arrays 33b is arranged between the second side 21b and the element array 22 outline. The first terminal arrays 33a can be formed in one line in parallel to the first side 21a. The second terminal arrays 33b can be formed in one line in parallel to the second side 21b. The first terminal array 33a is constituted by one pair of upper electrode terminals 34 and a plurality of lower electrode terminals 35. Similarly, the second terminal array 33b is constituted by a pair of upper electrode terminals 36 and a plurality of lower electrode terminals 37. The upper electrode terminals 34 and 36 are respectively connected to both ends of one lead-out wire 31. The lead-out wire 31 and the upper electrode terminals 34 and 36 can be formed plane-symmetrically at the perpendicular plane that bisects the element array 22. The lower electrode terminals 35 and 37 are respectively connected to both ends of one second conductor 32. The second conductor 32 and the lower electrode terminals 35 and 37 can be formed plane-symmetrically at the perpendicular plane that bisects the element array 22. Here, the outline of the base 21 is formed as a rectangle. The outline of the base 21 can also be square or can be a trapezoid.

A first flexible printed wiring board (hereafter called "first wiring board") 38 is coupled to the base 21. The first wiring board 38 is covered by the first terminal array 33a. A conductive line, specifically a first signal line 39, corresponding individually to the upper electrode terminal 34 and the lower electrode terminal 35, is formed on one end of the first wiring board 38. The first signal line 39 is bonded separately facing to individually match the upper electrode terminal 34 and the lower electrode terminal 35. Similarly, a second flexible printed wiring board (hereafter called "second wiring board") 41 is covered on the base 21. The second wiring board 41 is covered by the second terminal array 33b. A conductive line, specifically, a second signal line 42, is formed corresponding individually to the upper electrode terminal 36 and the lower electrode terminal 37 at one end of the second wiring board 41. The second signal line 42 is bonded separately facing to individually match the upper electrode terminal 36 and the lower electrode terminal 37.

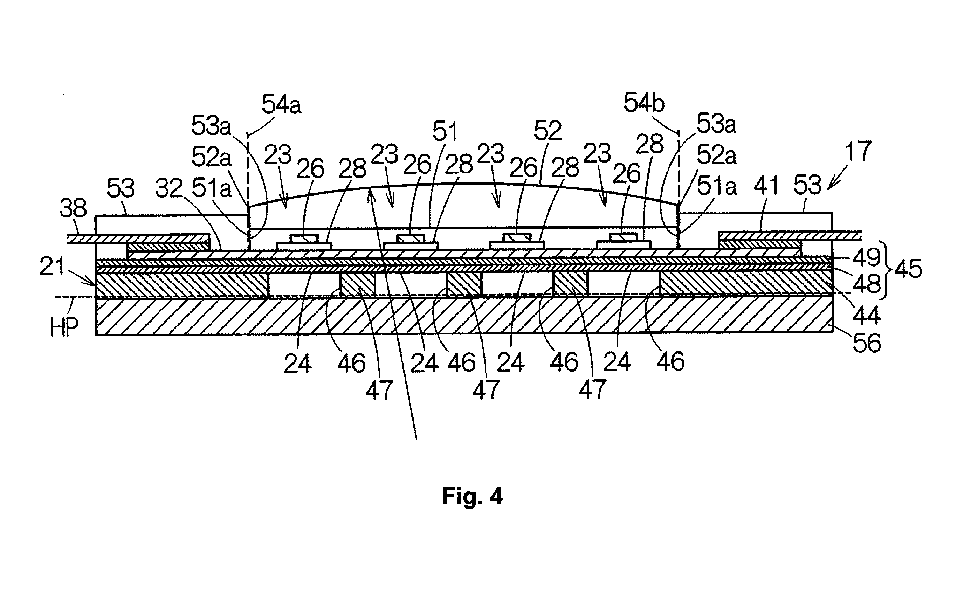

As shown in FIG. 4, the base 21 is equipped with a substrate 44 and a coating film 45. The coating film 45 is formed over the entire surface on the surface of the substrate 44. An opening 46 is formed for each individual element 23 on the substrate 44. The openings 46 are arranged in array form on the substrate 44. The outline of the area in which the openings 46 are arranged correlates to the outline of the element array 22. A partition wall 47 is demarcated between two adjacent openings 46. Adjacent openings 46 are partitioned by the partition wall 47. The wall thickness of the partition wall 47 correlates to the gap between the openings 46. The partition wall 47 defines two wall surfaces within the plane mutually extending in parallel. The wall thickness correlates to the distance between two wall surfaces. Specifically, the wall thickness can be regulated by the length of the perpendicular line sandwiched between the wall surfaces orthogonal to the wall surface. The substrate 44 can be formed with a silicon substrate, for example.

The coating film 45 is constituted by a silicon oxide (SiO₂) layer 48 laminated on the surface of the substrate 44, and a zirconium oxide (ZrO₂) layer 49 laminated on the surface of the silicon oxide layer 48. The coating film 45 is in contact with the opening 46. In this way, a portion of the coating film 45 corresponding to the outline of the opening 46 forms the vibration film 24. Of the coating film 45, the vibration film 24 is the part for which it is possible to do film vibration in the thickness direction of the substrate 44 since it faces the opening 46. The film thickness of the silicon oxide layer 48 can be determined based on the resonance frequency.

On the surface of the vibration film 24 are laminated in sequence the lower electrode 27, the piezoelectric film 28, and the upper electrode 26. The piezoelectric film 28 can be formed using lead zircon titanate (PZT), for example. It is also possible to use another piezoelectric material for the piezoelectric film 28. Here, the piezoelectric film 28 completely covers the surface of the second conductor 32 below the first conductor 29. It is possible to avoid shorting between the first conductor 29 and the second conductor 32 by the working of the piezoelectric film 28.

An acoustic adjustment layer 51 is laminated on the surface of the base 21. The acoustic adjustment layer 51 covers the element array 22. The film thickness of the acoustic adjustment layer 51 is determined according to the resonance frequency of the vibration film 24. It is possible to use a silicone resin film for the acoustic adjustment layer 51, for example. The acoustic adjustment layer 51 is housed in the space between the first terminal array 33a and the second terminal array 33b. The edge of the acoustic adjustment layer 51 is separated from the first side 21 a and the second side 21 b of the base 21. The acoustic adjustment layer 51 has an outline that is smaller than the outline of the base 21.

An acoustic lens 52 is arranged on the acoustic adjustment layer 51. The outline of the acoustic lens 52 overlaps the outline of the acoustic adjustment layer 51. Therefore, the edge of the acoustic lens 52 is separated from the first side 21a and the second side 21b of the base 21. The acoustic lens 52 is tightly adhered to the surface of the acoustic adjustment layer 51. The acoustic lens 52 is adhered to the base 21 by the working of the acoustic adjustment layer 51. The outer surface of the acoustic lens 52 is formed with a partial cylindrical surface. The partial cylindrical surface has a generatrix parallel to the first conductor 29. The curvature of the partial cylindrical surface is determined according to the focus position of the ultrasonic waves emitted from one row of elements 23 connected to one line of the second conductors 33. The acoustic lens 52 is formed from silicone resin, for example. The acoustic lens 52 has acoustic impedance close to the acoustic impedance of a living body.

A protective film (structural member) 53 is fixed to the base 21. The protective film 53 is formed from a material having water shielding properties such as epoxy resin, for example. However, it is also possible for the protective film 53 to be formed from another resin material. The protective film 53 has a modulus of rigidity that is greater than the modulus of rigidity of the acoustic lens 52. The protective film 53 is in contact with the acoustic lens 52 and the acoustic adjustment layer 51. Here, the protective film 53 is adhered to the side surfaces 52a and 51a of the acoustic lens 52 and the acoustic adjustment layer 51. The side surfaces 52a and 51a stand up from the surface of the base 21 perpendicular to the surface of the base 21. In FIG. 4, the protective film 53 sandwiches the acoustic lens 52 and the acoustic adjustment layer 51 with a contact surface 53a that extends in parallel to the generatrix of the acoustic lens 52 respectively along two virtual planes 54a and 54b intersecting the base 21 at a right angle. At this time, the side surfaces 52a and 51a of the acoustic lens 52 and the acoustic adjustment layer 51 are flush with each other. The protective film 53 covers the second conductor 32 and the lead-out wire 31 of the surface of the base 21 between the acoustic adjustment layer 51 and the first and second wiring boards 38 and 41. Similarly, the protective film 53 covers the end parts of the first wiring board 38 and the second wiring board 41 on the base 21.

A backing material 56 is fixed to the back surface of the base 21. The back surface of the base 21 is overlapped on the surface of the backing material 56. The backing material 56 closes the opening 46 on the back surface of the ultrasonic device 17. The backing material 56 can be equipped with a rigid base material. Here, the partition wall 47 is bonded to the backing material 56. The backing material 56 is bonded at a bonding area of at least one location on each partition wall 47. An adhesive agent can be used for the bonding.

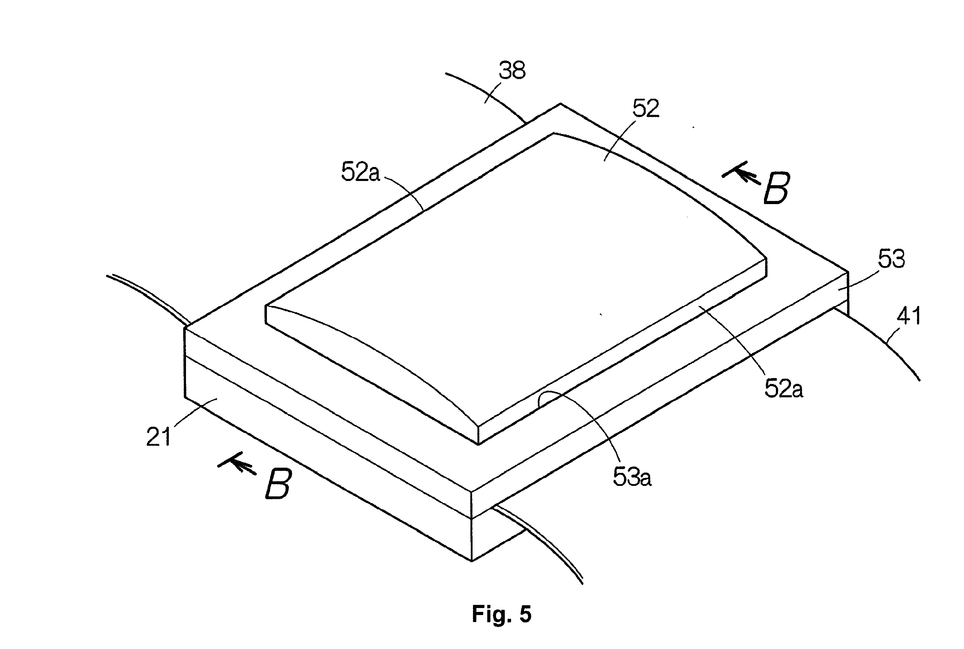

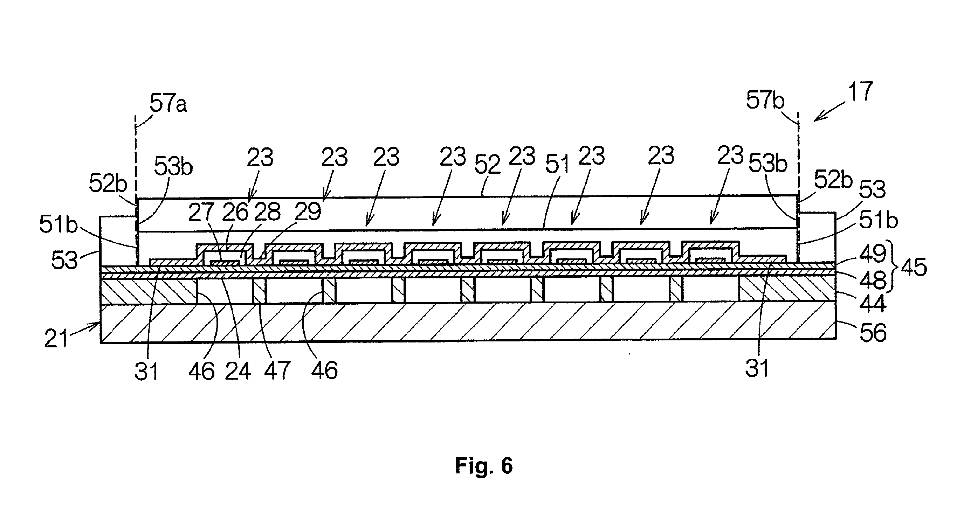

As shown in FIG. 5, the protective film 53 encloses the acoustic lens 52 along the outline of the acoustic lens 52 in a plan view. The protective film 53 extends in a frame shaped between the outline of the acoustic lens 52 and the peripheral edge of the base 21. As shown in FIG. 6, when forming the frame shape, the protective film 53 is adhered to the side surfaces 52b and 51b of the acoustic lens 52 and the acoustic adjustment layer 51. The side surfaces 52b and 51b stand up from the surface of the base 21 perpendicular to the base 21, and connection is done perpendicular to the side surfaces 52b and 51b. The protective film 53 sandwiches the acoustic lens 52 and the acoustic adjustment layer 51 with contact surface 53b respectively along the two virtual planes 57a and 57b that intersects the base 21 at a right angle while intersecting the generatrix of the acoustic lens 52 at a right angle. At this time, the side surfaces 52b and 51b of the acoustic lens 52 and the acoustic adjustment layer 51 are flush with each other.

### (2) Operation of the Ultrasonic Diagnostic Device

Next, we will give a brief description of the operation of the ultrasonic diagnostic device 11. For sending of ultrasonic waves, pulse signals are supplied to the piezoelectric element 25. The pulse signals are supplied to the elements 23 for each row through the lower electrode terminals 35 and 37 and the upper electrode terminals 34 and 36. With each element 23, an electric field acts on the piezoelectric film 28 between the lower electrode 27 and the upper electrode 26. The piezoelectric film 28 vibrates with the frequency of the ultrasonic waves. The vibration of the piezoelectric film 28 is conveyed to the vibration film 24. In this way, the vibration film 24 does ultrasonic vibration. As a result, the desired ultrasonic beams are emitted toward the subject (e.g. the interior of a human body).

The reflected waves of the ultrasonic waves vibrate the vibration film 24. The ultrasonic vibration of the vibration film 24 makes the piezoelectric film 28 do ultrasonic vibration at a desired frequency. Voltage is output from the piezoelectric element 25 according to the piezoelectric effect of the piezoelectric element 25. Electric potential is generated between the upper electrode 26 and the lower electrode 27 with each element 23. The electric potential is output as electric signals from the lower electrode terminals 35 and 37 and the upper electrode terminals 34 and 36. In this way, ultrasonic waves are detected.

The sending and receiving of ultrasonic waves is repeated. As a result, linear scanning or sector scanning is realized. When scanning is completed, an image is formed based on the digital signals of the output signals. The formed image is displayed on the screen of the display panel 15.

When forming the ultrasonic image, the acoustic lens 52 is pressed against a living body. When the acoustic lens 52 is moved along the surface of the living body in the direction orthogonal to the generatrix of the acoustic lens 52, the acoustic lens 52 is exposed to shear force from the direction orthogonal to the generatrix of the acoustic lens 52. At this time, the side surfaces 52a and 51a of the acoustic lens 52 and the acoustic adjustment layer 51 are received by the protective film 53 in the shearing direction. Even when shear force is imparted to the acoustic lens 52, it is possible to prevent shear deformation of the acoustic lens 52. In this way, with the ultrasonic device 17, it is possible to sufficiently suppress shear deformation of the acoustic lens 52 in relation to the shear force. Prevention of shear deformation of the acoustic adjustment layer 51 can greatly contribute to the suppression of shear deformation of the acoustic lens 52.

As described previously, a partial cylindrical surface formed by a generatrix parallel to one center line is defined on the acoustic lens 52. The partial cylindrical surface is used to converge ultrasonic waves. The focus position of the ultrasonic waves is determined by the partial cylindrical surface. The contact surface 53a of the protective film 53 sandwiches the acoustic lens 52 in the direction intersecting at a right angle with the generatrix, so shear deformation of the acoustic lens 52 is prevented in the direction intersecting with the generatrix. In this way, it is possible to maintain the focus position of the acoustic lens 52. When the acoustic lens 52 has shear deformation in the direction intersecting the generatrix of the acoustic lens 52, there is a risk of displacement of the focus position of the acoustic lens 52 occurring. When the focus position is displaced, it is not possible to draw an accurate ultrasonic image. In fact, with this embodiment, the protective film 53 sandwiches the acoustic lens 52 with the contact surface 53b in the direction along the generatrix of the acoustic lens 52. Shear deformation of the acoustic lens 52 is prevented in the direction along the generatrix. Preventing shear deformation in this way can greatly contribute to prevention of displacement of the focus position.

With the ultrasonic device 17, the side surfaces 52a and 51 a of the acoustic lens 52 and the acoustic adjustment layer 51 are respectively adhered to the contact surface 53a which is a single plane. Therefore, the side surfaces 52a and 51a of the acoustic lens 52 and the acoustic adjustment layer 51 are flush with each other. As will be described later, during production of the ultrasonic device 17, a solid acoustic lens 52 is adhered to the element array 22 using a fluid resin material. The resin material is hardened to form the acoustic adjustment layer 51. At this time, on the base 21, at the same time that the acoustic lens 52 edge is being aligned in the direction intersecting at a right angle the generatrix of the acoustic lens 52, a physical surface is formed that dams the flow of the resin material. The acoustic lens 52 is accurately aligned to the element array 22 in the direction intersecting the generatrix at a right angle. When the resin material hardens, the side surface 52a of the acoustic lens 52 and the side surface 51a of the acoustic adjustment layer 51 are flush with each other. Similarly, the side surfaces 52b and 51b of the acoustic lens 52 and the acoustic adjustment layer 51 are respectively adhered to the contact surface 53b that is a single plane. Therefore, the side surfaces 52b and 51b of the acoustic lens 52 and the acoustic adjustment layer 51 are flush with each other. During production of the ultrasonic device 17, on the base 21, at the same time that the acoustic lens 52 edge is being aligned in the direction parallel to the generatrix of the acoustic lens 52, a physical surface is formed that dams the flow of the resin material. The acoustic lens 52 is accurately aligned to the element array 22 in the direction parallel to the generatrix. When the resin material hardens, the side surface 52b of the acoustic lens 52 and the side surface 51b of the acoustic adjustment layer 51 are flush with each other.

With the ultrasonic device 17, the element array 22 and the first and second wiring boards 38 and 41 are electrically connected to each other by a second conductor 33 and a lead-out wire 31 on the base 21. The protective film 53 is covered on the second conductor 32 and the lead-out wire 31 on the base 21 between the acoustic adjustment layer 51 and the first and second wiring boards 38 and 41. In this way, the second conductor 33 and the lead-out wire 31 are covered by the protective film 53 between the acoustic adjustment layer 51 and the first and second wiring boards 38 and 41, so exposure of the second conductor 33 and the lead-out wire 31 is avoided. In this way, the conductor on the base 21 is protected. In particular, when water shielding properties are given to the protective film 53, the second conductor 33 and the lead-out wire 31 are protected from moisture and humidity, and it is possible to prevent short circuits between conductive materials such as the second conductor 33 and the lead-out wire 31. As described previously, when the protective film 53 is covered on the end parts of the first and second wiring boards 38 and 41, it is possible for the protective film 53 to reinforce the fixing strength of the first and second wiring boards 38 and 41.

### (3) Ultrasonic Device Manufacturing Method

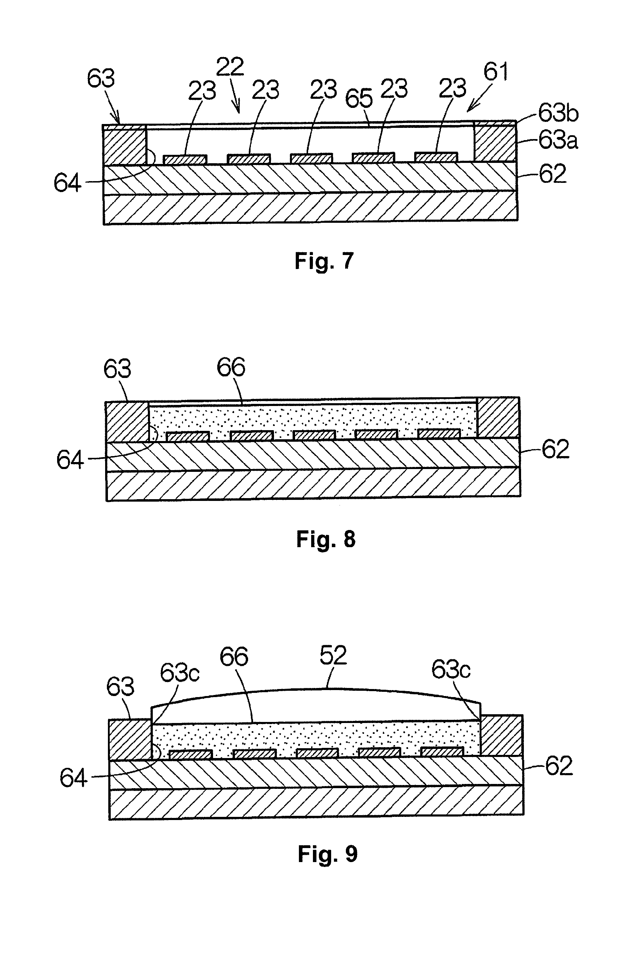

Next, we will describe the manufacturing method of the ultrasonic device 17. As shown in FIG. 7, a substrate 61 is prepared. The substrate 61 has the element array 22 including a plurality of elements 23 arranged in array form on a base material 62. The base material 62 correlates to the base 21 described previously. A masking material 63 is laminated on the base material 62. The masking material 63 is formed in an enclosure shape between the outline of the element array 22 and the peripheral edge of the base material 62 in the plan view. The masking material 63 has an opening 64 formed on the element array 22. The opening 64 demarcates a frame enclosing the element array 22 on the base material 62. Therefore, at the same time that the masking material 63 is arranged at both sides of the outline of the element array 22 in the direction in which the second conductor 33 extends in a plan view, it is arranged at both sides of the outline of the element array 22 in the direction in which the first conductor 29 extends. Here, the masking material 63 is formed into a two layer laminated structure. The thickness of the lower layer 63a matches the film thickness of the acoustic adjustment layer 51. An interface 65 of the lower layer 63a and the upper layer 63b can function as a marker indicating the film thickness of the acoustic adjustment layer 51. It is possible to use a metal mask or photo resist for the masking material 63.

Subsequently, the acoustic adjustment layer 51 and the acoustic lens 52 are arranged inside the opening 64 of the masking material 63. When doing these arrangements, as shown in FIG. 8, the resin material 66 of the acoustic adjustment layer 51 flows into the opening 64 of the masking material 63. The flow of the resin material 66 is dammed by the masking material 63. In this way, the shape of the acoustic adjustment layer 51 is arranged. The resin material 66 uniformly extends inside the opening 64 of the making material 63. Here, the volume of the resin material 66 is adjusted based on the marker. The thickness of the resin material 66 can be controlled with high precision by the working of the marker.

As shown in FIG. 9, the acoustic lens 52 is overlapped on the resin material 66 inside the opening 64 of the masking material 63. The acoustic lens 52 is acceptable provided it is processed into a predetermined shape. The masking material 63 aligns the acoustic lens 52 from both sides in the extending direction of the second conductor 33 in a plan view, and at the same time, aligns the acoustic lens 52 from both sides in the extending direction of the first conductor 29. Therefore, the masking material 63 implements alignment from four sides on the acoustic lens 52 within the opening 64. The acoustic lens 52 can be aligned to the element array 22 with high precision by the working of the masking material 63. Here, the wall surface 63c of the masking material 63 is formed by a surface orthogonal to the surface of the base material 62. The edge of the acoustic lens 52 has surface contact with the wall surface 63c of the masking material 63. The resin material 66 is hardened in accordance with the irradiation of heat or ultraviolet light. The resin material 66 is hardened to form the acoustic adjustment layer 51. In this way, the acoustic lens 52 is adhered to the element array 22.

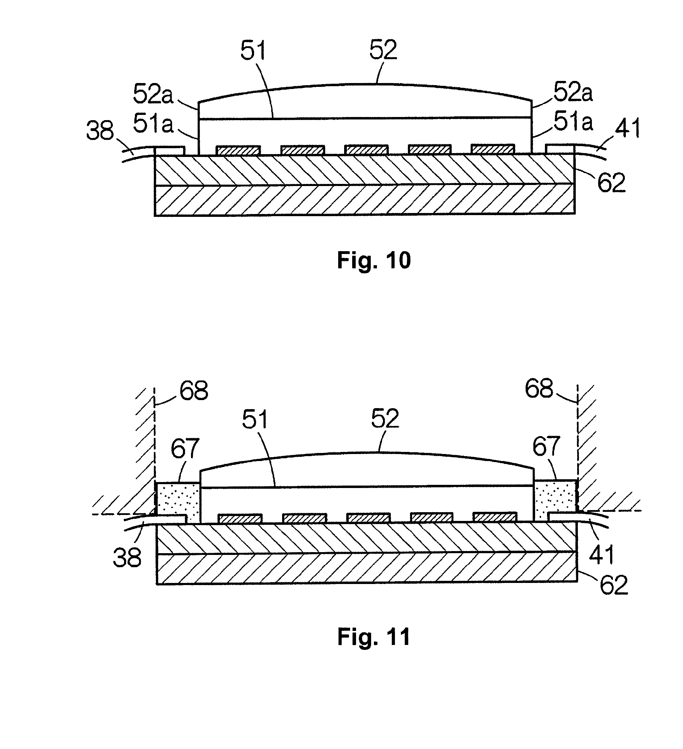

As shown in FIG. 10, when the acoustic adjustment layer 51 hardens, the masking material 63 is removed. The masking material 63 can be removed using an etching process, for example, or something else. As described previously, the edge of the acoustic lens 52 is in surface contact with the wall surface 63c of the masking material 63, so the side surface 52a of the acoustic lens 52 and the side surface 51a of the acoustic adjustment layer 51 are mutually flush with each other, and the side surface 52b of the acoustic lens 52 and the side surface 51b of the acoustic adjustment layer 51 are mutually flush with each other.

When the masking material 63 is removed, a first terminal array 33a and a second terminal array 33b are exposed between the outline of the element array 22 and the peripheral edge of the base material 62 on the surface of the base material 62. After the masking material 63 is removed, the first wiring board 38 and the second wiring board 41 are respectively coupled between the outline of the element array 22 and the peripheral edge of the base material 62. The first wiring board 38 is covered on the first terminal array 33a. The second wiring board 41 is covered on the second terminal array 33b. The first signal line 39 of the first wiring board 38 is individually connected to the upper electrode terminal 34 and the lower electrode terminal 35. The second signal line 42 of the second wiring board 41 is individually connected to the upper electrode terminal 36 and the lower electrode terminal 37. For bonding, pressure welding or another bonding method can be used.

After that, as shown in FIG. 11, the protective film 53 is formed on the base material 62. A resin material 67 is flowed in between the acoustic adjustment layer 51 and the first wiring board 38, and between the acoustic adjustment layer 51 and the second wiring board 41. For supplying of the resin material 67, for example a nozzle can be used. The nozzle can be moved along the edge of the first wiring board 38 and the edge of the second wiring board 41 in parallel with the generatrix of the acoustic lens 52. The resin material 67 has fluidity. For flowing in of the resin material 67, an enclosure 68 can be demarcated on the first wiring board 38 and the second wiring board 41. The enclosure 68 can be formed from a metal mold, for example. The enclosure 68 can dam the flow of the resin material 67. The resin material 67 is hardened in accordance with the irradiation of heat or ultraviolet light. The resin material 67 is hardened to form the protective film 53. Epoxy resin can also be used for the resin material 66 and the resin material 67. In that case, the acoustic lens 52 can also be formed from epoxy resin.

### (4) Ultrasonic Device of a Modification Example

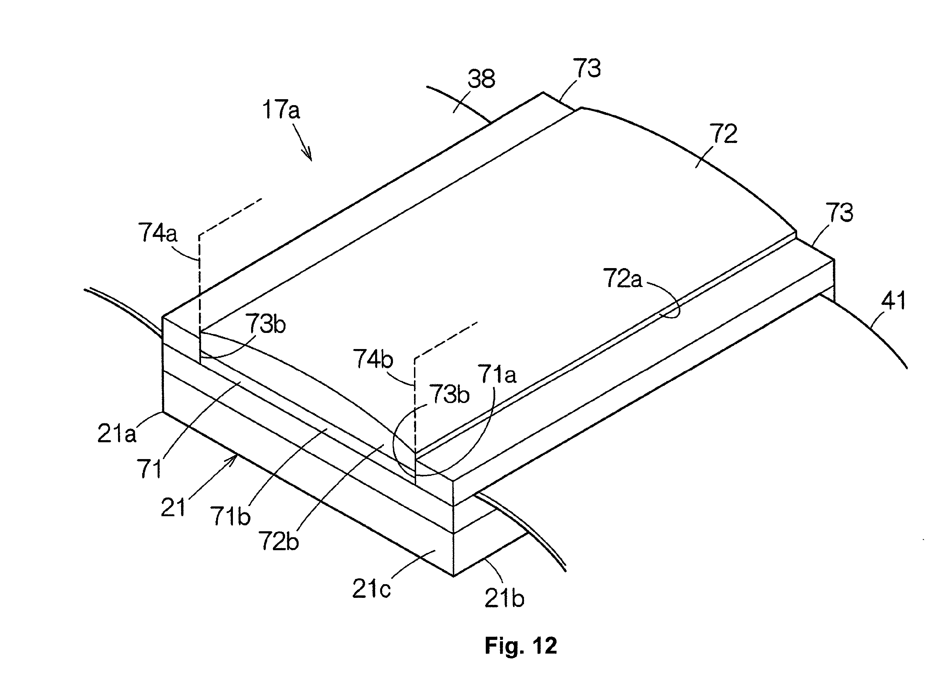

FIG. 12 schematically shows the ultrasonic device 17a of the first modification example. With the ultrasonic device 17a, an acoustic adjustment layer 71 and an acoustic lens 72 have an outline separated from the first side 21a and the second side 21b of the base 21. Therefore, the acoustic adjustment layer 71 and the acoustic lens 72 are formed to be smaller than the base 21 in the direction intersecting at a right angle with the generatrix of the acoustic lens 72. On the other hand, the acoustic adjustment layer 71 and the acoustic lens 72 extend to the same size as the base 21 in the direction parallel to the generatrix of the acoustic lens 72. Therefore, the acoustic adjustment layer 71 and the acoustic lens 72 extend to the maximum limit on the base 21 in the direction parallel to the generatrix. Side surfaces 72b and 71b of the acoustic lens 72 and the acoustic adjustment layer 71 are flush with the side surface 21c of the base 21. A protective film 73 extends in parallel to the acoustic adjustment layer 71 and the acoustic lens 72 in parallel with the generatrix of the acoustic lens 72. The protective film 73 extends in parallel to the generatrix of the acoustic lens 52 and sandwiches the acoustic lens 72 and the acoustic adjustment layer 71 with the contact surface 73a respectively along two virtual planes 74a and 74b intersecting at a right angle to the base 21. The rest of the constitution is the same as that of the previously described ultrasonic device 17.

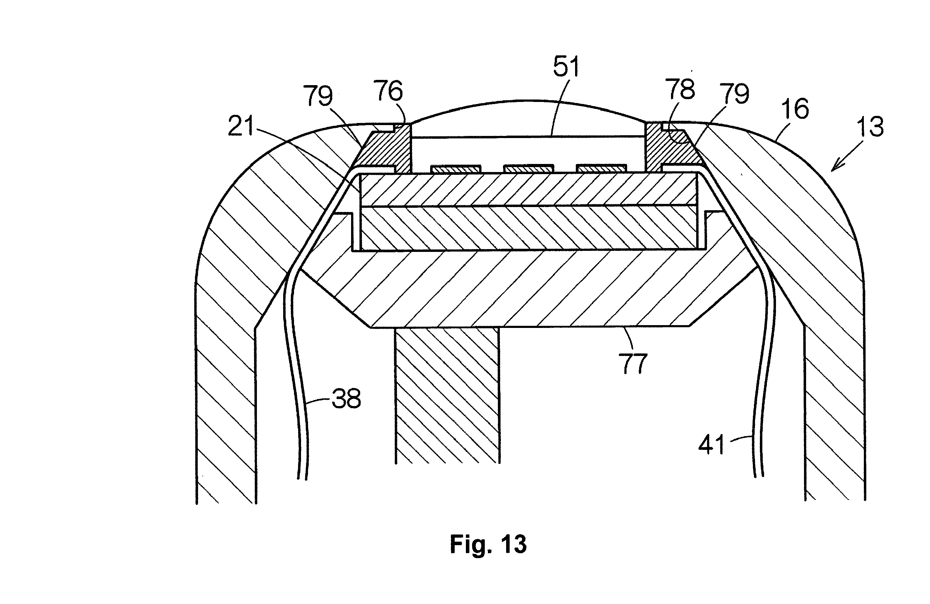

FIG. 13 schematically shows an ultrasonic device 17b of a second modification example. The case 16 of the ultrasonic probe 13 forms an opening 76. The acoustic lens 52 is arranged in the opening 76. The ultrasonic device 17b is supported on a support member 77. The support member 77 is coupled to the inside of the case 16. When coupling, the first wiring board 38 and the second wiring board 41 are sandwiched between the support member 77 and the case 16. On the periphery of the acoustic lens 52 and the acoustic adjustment layer 51, a space 78 is demarcated between the base 21 and the case 16. The space 78 is filled with a protective material 79. The protective material 79 has a modulus of rigidity greater than the modulus of rigidity of the acoustic lens 52. The protective material 79 fixes the acoustic lens 52, the acoustic adjustment layer 51, and the base 21 to the case 16. The protective material 79 can function in the same way as the protective film 53 described above. The remainder of the constitution is the same as the ultrasonic device 17 described previously.

When producing the ultrasonic probe 13, the ultrasonic device 17b and the support member 77 are housed in the case 16. When the support member 77 is fixed to the case 16, the acoustic lens 52 faces the opening 76. At this time, at the periphery of the acoustic lens 52 and the acoustic adjustment layer 51, the space 78 is demarcated between the base 21 and the case 16. A resin material having fluidity is filled in the space 78 from the gap of the opening 76. When the resin material is hardened, the protective material 79 is formed. The protective material 79 prevents movement of the acoustic lens 52 inside the opening 76. The acoustic lens 52 is reliably fixed to the case 16.

We gave a detailed description of the embodiments as noted above, but a person skilled in the art will easily understand that it is possible to have many modifications without substantially straying from the novel items and effects of the present invention. Therefore, all of these kinds of modification examples are included within the scope of the present invention. For example, for terminology noted at least once together with a different term having a broader or the same meaning in the specification or drawings, that different terminology can be used as a substitute in any location in the specification or drawings. Also, the constitution and operation of the ultrasonic diagnostic device 11, the device terminal 12, the ultrasonic probe 13, the display panel 15, the case 16, the base 21, the elements 23, the first and second wiring boards 38 and 41, the acoustic adjustment layer 51 and 71, the acoustic lens 52 and 72 and the like are not limited to the items described with the embodiments, but can also have various modifications.

### GENERAL INTERPRETATION OF TERMS

In understanding the scope of the present invention, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives. Also, the terms "part," "section," "portion," "member" or "element" when used in the singular can have the dual meaning of a single part or a plurality of parts. Finally, terms of degree such as "substantially", "about" and "approximately" as used herein mean a reasonable amount of deviation of the modified term such that the end result is not significantly changed. For example, these terms can be construed as including a deviation of at least ± 5% of the modified term if this deviation would not negate the meaning of the word it modifies.

While only selected embodiments have been chosen to illustrate the present invention, it will be apparent to those skilled in the art from this disclosure that various changes and modifications can be made herein without departing from the scope of the invention as defined in the appended claims. Furthermore, the foregoing descriptions of the embodiments according to the present invention are provided for illustration only, and not for the purpose of limiting the invention as defined by the appended claims and their equivalents.

## Claims

1. An ultrasonic device comprising:
a substrate having an element array including a plurality of thin film ultrasonic transducer elements arranged in an array form;
an acoustic adjustment layer covering the element array;
an acoustic lens arranged above the acoustic adjustment layer; and
a structural member in contact with the acoustic lens and fixed to the substrate, the structural member having a modulus of rigidity greater than a modulus of rigidity of the acoustic lens.

2. The ultrasonic device according to claim 1, wherein
the acoustic adjustment layer is in contact with the structural member, and is fixed to the substrate.

3. The ultrasonic device according to claim 1 or 2, wherein
the structural member includes a pair of side surfaces that extend in parallel with a generatrix of the acoustic lens, the side surfaces of the structural member being respectively in contact with side surfaces of the acoustic lens as the acoustic lens is sandwiched by the structural member.

4. The ultrasonic device according to any one of the preceding claims, wherein
the acoustic adjustment layer has a pair of side surfaces that extend flush with the side surfaces of the acoustic lens, respectively.

5. The ultrasonic device according to any one of claims 1 to 3, wherein
the structural member includes a pair of additional side surfaces that extend in a direction intersecting with the generatrix of the acoustic lens, the additional side surfaces of the structural member being respectively in contact with additional side surfaces of the acoustic lens as the acoustic lens is sandwiched by the structural member.

6. The ultrasonic device according to claim 5, wherein
the acoustic adjustment layer has a pair of additional side surfaces that extend flush with the additional side surfaces of the acoustic lens, respectively.

7. The ultrasonic device according to claim 4, wherein
the acoustic lens has a pair of additional side surfaces that extend in a direction intersecting the generatrix of the acoustic lens,
the acoustic adjustment layer includes a pair of additional side surfaces that extend in the direction intersecting the generatrix of the acoustic lens, and
the additional side surfaces of the acoustic lens and the additional side surfaces of the acoustic adjustment layer are flush with a pair of side surfaces of the substrate, respectively.

8. The ultrasonic device according to any one of the preceding claims, further comprising:
a flexible printed wiring board coupled to the substrate at an outside of an outline of the acoustic adjustment layer in a plan view along a thickness direction of the substrate; and
a conductor member disposed on the substrate, wherein
at least a part of the structural member is arranged on the conductor member between the acoustic adjustment layer and the flexible printed wiring board.

9. The ultrasonic device according to claim 8, wherein
at least a part of the structural member is arranged on the flexible printed wiring board.

10. A probe comprising:
the ultrasonic device according to any one of the preceding claims; and
a case supporting the ultrasonic device.

11. The probe according to claim 10, wherein
the structural member is fixed to the case.

12. An electronic equipment comprising:
the ultrasonic device according to any one of claims 1 to 9; and
a processing unit connected to the ultrasonic device, and configured to process an output of the ultrasonic device.

13. An ultrasonic image device comprising:
the ultrasonic device according to any one of claims 1 to 9;
a processing unit connected to the ultrasonic device, and configured to process an output of the ultrasonic device to generate an image; and
a display device configured and arranged to display the image.

14. An ultrasonic device manufacturing method comprising:
arranging a masking material on a substrate having an element array including a plurality of thin film ultrasonic transducer elements arranged in an array form so that both sides of an area on which the element array is arranged in a plan view along a thickness direction of the substrate are covered by the masking material;
arranging an acoustic adjustment layer and an acoustic lens on the element array between the masking material;
removing the masking material; and
forming a structural member in contact with the acoustic lens and fixed to the substrate, the structural member having a modulus of rigidity greater than a modulus of rigidity of the acoustic lens.

15. The ultrasonic device manufacturing method according to claim 14, further comprising
after the removing of the masking material, coupling a flexible printed wiring board to the substrate between edges of the area and edges of the substrate in the both sides of the area, wherein
the forming of the structural member includes flowing a resin material having fluidity in between the acoustic adjustment layer and the flexible printed wiring board, and hardening the resin material to form the structural member.

16. The ultrasonic device manufacturing method according to claim 14 or 15,
wherein
the arranging of the acoustic lens includes using the masking material to position the acoustic lens from both sides.

17. The ultrasonic device manufacturing method according to claim 16, wherein
the arranging of the acoustic lens includes placing the acoustic lens within an opening formed in the masking material to position the acoustic lens from four directions on the acoustic lens.

18. The ultrasonic device manufacturing method according to claim 17, wherein
the arranging of the acoustic adjustment layer includes flowing a resin material having fluidity into the opening and controlling a thickness of the resin material by a thickness of the masking material.
